# EUROPEAN PATENT SPECIFICATION

(11) **EP 1 712 625 B1**
(45) Date of publication and mention of the grant of the patent: **04.08.2010**
(21) Application number: 04734498.1
(22) Date of filing: 24.05.2004
(51) Int. Cl.: C12N 15/29, C12N 15/82

(54) **AN ARABIDOPIS THALIANA TRANSCRIPTION FACTORAS WELL AS THE ENCODING GENE AND THE USE THEREOF**
TRANSKRIPTIONSFAKTOR AUS ARABIDOPIS THALIANA SOWIE CODIERENDES GEN UND VERWENDUNG DAVON
FACTEUR DE TRANSCRIPTION ARABIDOPIS THALIANA, GENE LE CODANT ET UTILISATION CORRESPONDANTE

(30) Priority: 15.01.2004 CN 200410006622
(43) Date of publication of application: 18.10.2006
(73) Proprietor: Xiang, Chengbin, Hefei Anhui 230026 (CN)
(72) Inventor: Xiang, Chengbin, Hefei Anhui 230026 (CN)
(74) Representative: Ziebig, Marlene
(86) International application number: PCT/CN2004/000524
(87) International publication number: WO 2005/071083

(56) References cited:
- WO-A-2004/031349
- WO-A2-03/013227
- DATABASE GENBANK [Online] 04 March 2003 YAMADA K. ET AL Database accession no. (BT00)915

## Description

### Technical field

This invention relates to the use of the *Arabidopsis thaliana* transcription factor gene *AtHD*/*START1* encoding a plant transcription factor in the field of gene engineering for crop improvement of plants with drought stress tolerance.

### Background of the invention

During the growth and development of plants, any unfavorable environmental conditions can be considered as environmental stresses, generally divided into biotic stress and abiotic stress. Abiotic stress is caused by unfavorable conditions of water, temperature, salt, light, nutrition, wind, and the like.

The negative impact that the environmental stresses bring to the agricultural production is worldwide, and the impact caused by the abiotic stress, especially drought, is even worse (Boyer JS. 1982. Plant productivity and environment. Science 218:443-448). As Table 1 shows, abiotic stresses can reduce crop yields by 50% to 80%. Just because of this tremendous potential for profit in agriculture, huge efforts have been poured into the research of abiotic stress biology worldwide in order to discover key abiotic stress tolerance genes and unravel the molecular mechanisms underlying stress tolerance for crop improvement by gene engineering.

**Table 1: The average yield and the record high yield of eight different crops**

| Crop | The record high yield (kg/ha) | The average yield (kg/ha) | The average loss (kg/ha) | | The loss caused by abiotic stress (% of record high yield) |
|---|---|---|---|---|---|
| | | | Biotic stress | Abiotic stress | |
| maize | 19,300 | 4,600 | 1,952 | 12,700 | 65.8 |
| wheat | 14,500 | 1,880 | 726 | 11,900 | 82.1 |
| soybean | 7,390 | 1,610 | 666 | 5,120 | 69.3 |
| broomcorn | 20,100 | 2,830 | 1,051 | 16,200 | 80.6 |
| oat | 10,600 | 1,720 | 924 | 7,960 | 75.1 |
| barley | 11,400 | 2,050 | 765 | 8,590 | 75.4 |
| potato | 94,100 | 28,300 | 17,775 | 50,900 | 54.1 |
| beet | 121,000 | 42,600 | 17,100 | 61,300 | 50.7 |

| | | | | | |
|---|---|---|---|---|---|
| Note: Biotic stress includes diseases, insects, and weeds; abiotic stress mainly includes drought, salt, waterlog, low temperature and high temperature. | | | | | |

Agriculture occupies a large sector in the economy of China, but abiotic stresses are major threats of Chinese agriculture, especially drought and salt stress being the two most important factors limiting sustainable agriculture. In China, 50% of arable farmland is affected by drought. Even in central and southern China where have more rainfall, drought still occurs during the reproductive phase of rice, the predominant crop in the region because of the uneven distribution of the rainfall, and causes tremendous yield loss. If encountered severe drought, the whole crop yield may be lost. Insufficient rainfall in the vast area of northern and northwestern China makes the soil salinity problem even worse, which has become one of the major restraints of sustainable agriculture in the region.

The research of abiotic stress biology and cloning of stress tolerance gene are thus very important and urgent. Plant biologists worldwide work very hard to unravel the molecular mechanisms and isolate stress tolerance genes for crop improvement. Progress has been made, especially in the field of the molecular mechanism of salt stress by using the model plant *Arabidopsis thaliana.* Recently there were many important new discoveries (Zhu JK. 2002. Salt and drought stress signal transduction in plants. Annu. Rev. Plant Biol. 53:1247-273 ; Xiong LM, Schumaker KS, Zhu JK. 2002. Cell signaling during cold, drought, and salt stress. Plant Cell, S 165-S 183). For higher plants, sophisticated mechanisms have evolved to perceive the physical and chemical changes in their surrounding environment, and respond correspondingly by transducing the extracellular signals into the intracellular signals, and eventually relay the signals into nucleus and activate transcription factors that turn on corresponding gene expression, deploying their defense arsenals and adjusting their growth and development in order to adapt to the environmental changes. The research on plant drought stress has been focused on the aspects of osmoregulation and the signal transduction of abscisic acid (ABA) and the loss-of-function mutants of *Arabidopsis thaliana* played an important role to this research (Zhu JK. 2002. Salt and drought stress signal transduction in plants. Annu. Rev. Plant Biol. 53:1247-273 ; Xiong LM, Schumaker KS, Zhu JK. 2002. Cell signaling during cold, drought, and salt stress. Plant Cell. S165-S183). In contrast to the loss-of-function mutant, the gain-of-function mutants have not been adequately explored although the mutants can provide valuable materials for stress tolerance gene discovery and the cloned gene can be directly used for crop improvement.

### Detailed description of the invention

Therefore, it is an object of the invention to provide plants with crop improvement.

Subject matter of the invention is the use of the transcription factor gene named as *AtHD*/*START1* (or *HS1* in short) for improving drought tolerance of plants. The gene is originated from the *Arabidopsis thaliana* mutant *hs1*, and includes one of the following nucleotide sequences:
1) a nucleotide sequence of SEQ ID Nº: 1;
2) a nucleotide sequence encoding the amino acid sequence of SEQ ID N₂: 2;
3) a nucleotide sequence having more than 80% homology with the nucleotide sequence of SEQ ID N₂: 1 with the same or similar biological functions.

The sequence ofcDNA in the SEQ ID N₂: 1 is composed of 2169 bases, the open reading frame (ORF) of which begins at 1^{st} base of 5' end and ends at 2169^{th} base.

The transcription factor *AtHD*/*START1,* encodes a protein with the sequence of amino acid residues in SEQ ID Nº: 2 of the sequence listing, or is any protein derived from the protein of SEQ ID Nº: 2 which has one or several amino acid residues substituted, deleted or inserted but still has the same biological functions.

The sequence of amino acid residues for the protein as shown in SEQ ID Nº: 2 is made up of 722 amino acid residues.

By using any suitable vectors and feasible means of transformation for heterologous expression, the *AtHD*/*START1* gene is integrated in the recipient plant genome and overexpressed in the transgenic plants. As a result, the transgenic plants can confer improved tolerance to drought stress. In contrast, when the *AtHD*/*START1* gene was knocked-out, the plants will not exhibit the properties of tolerance to drought stress. In order to select and identify the transformed plant cells and transgene plants, appropriate marker genes such as antibiotic resistance genes can be added to the construct vectors. The host plants can be either monocots or dicots such as rice, wheat, maize, cucumber, tomato, aspen, grass, clover and so on.

### Description of the Drawings

### (Figures 3 to 4 ant 7 to 13 are no part of the present invention)

Figure 1a shows the morphological characteristics of the aboveground portion of the gain-of-function mutant.
Figure 1b shows the comparison of the length of primary root between the gain-of-function mutant and the wildtype.
Figure 1c shows the comparison of the number of lateral roots between the gain-of-function mutant and the wildtype.
Figure 1d shows data for comparison of the number of lateral roots and the biomass of root system between the gain-of-function mutant and the wildtype.
Figure 2a shows the responses of the wild type and the mutant to drought stress.
Figure 2b shows the response of the T2 population to drought stress
Figure 2c shows the drought stress assay of the wildtype and the mutant in the same pot.
Figure 2d shows the comparison of the water loss rate of detached leaves in the air between the wild type and the mutant.
Figure 3a shows the comparison between the wild type and the mutant in response to salt stress of 0.2M NaCl after 18 days.
Figure 3b shows the comparison between the wild type and the mutant seedlings in response to salt stress.
Figure 3c shows the data for comparison between the wild type and the mutant seedlings in response to salt stress.
Figure 4 shows the comparison between the wild type and the mutant seedlings to oxidation stress.
Figure 5 shows the analysis of the expression of the tagged gene in the rosette leaves of the mutant T2 vs. the wildtype by RT-PCR.
Figure 6 illustrates the functional domains of *Arabidopsis thaliana HD*/*START1.*

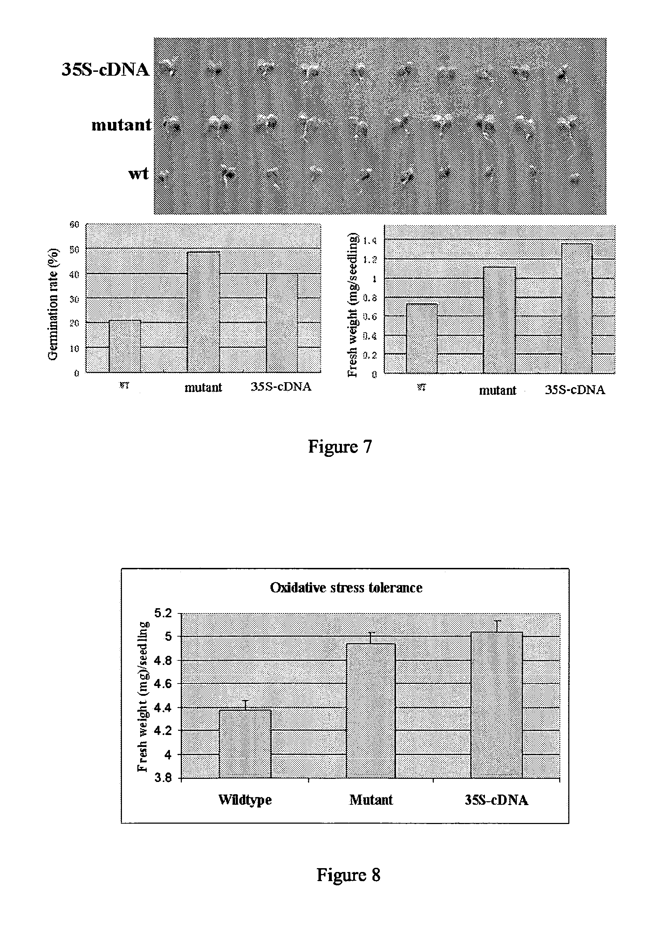

Figure 7 shows the germination of the 35S-cDNA transformant, the mutant, and the wild type seeds under salt stress (150mM NaCl).
Figure 8 shows the comparison of response to oxidative stress (paraquat treatment) between the 35S-cDNA transformant, the mutant, and the wild type.

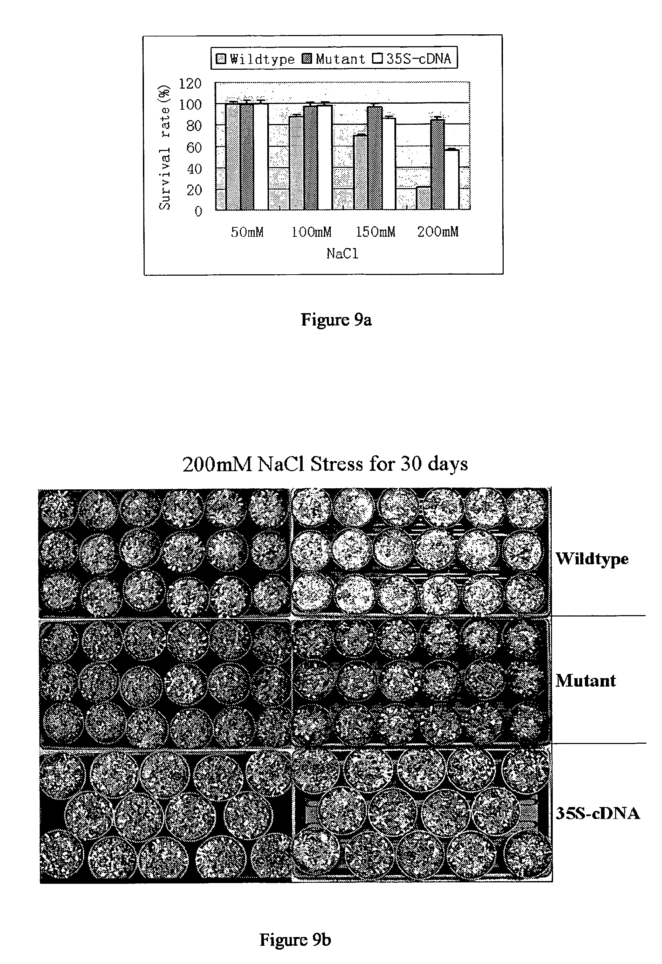

Figure 9a shows the data for comparison of survival ratio between the 35S-cDNA transformant, the mutant, and the wild type under different salt concentrations.
Figure 9b shows the comparison of survival ratio between the 35S-cDNA transformant, the mutant, and the wild-type under 200mM NaCl salt stress for 30 days.

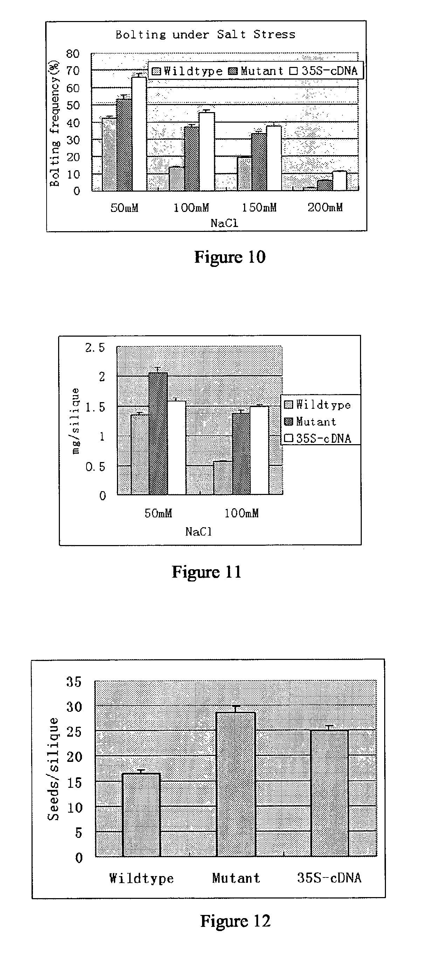

Figure 10 shows the data for comparison of bolting frequency between the wild type, the mutant, and the 35S-cDNA transformant under different salt concentrations.
Figure 11 shows the data for comparison of silique weight between the wild type, the mutant, and the 35S-cDNA transformant under different salt concentrations.
Figure 12 shows the data for comparison of silique seed yield between the wild type, the mutant, and the 35S-cDNA transformant under 100mM NaCl stress.
Figure 13 shows the comparison silique and plant between the wild type, the mutant, and the 35S-cDNA transformant under 100mM NaCl stress.
Figure 14 shows the comparison of the drought response between the 35S-cDNA transformant and the wild type.

### Detailed description of the preferred embodiment

### Example 1. The screening, phenotypic characterization of the gain-of-function mutant.

For isolating the gain-of-function mutant, the inventor generated an *Arabidopsis thaliana* T-DNA activation tagging mutant library of ∼55,000 independent lines. (Weigel et al., 2000. Activation tagging in Arabidopsis. Plant Physio1.122:1003-13.), and isolated a few mutants with enhanced growth vigor and stress tolerance.

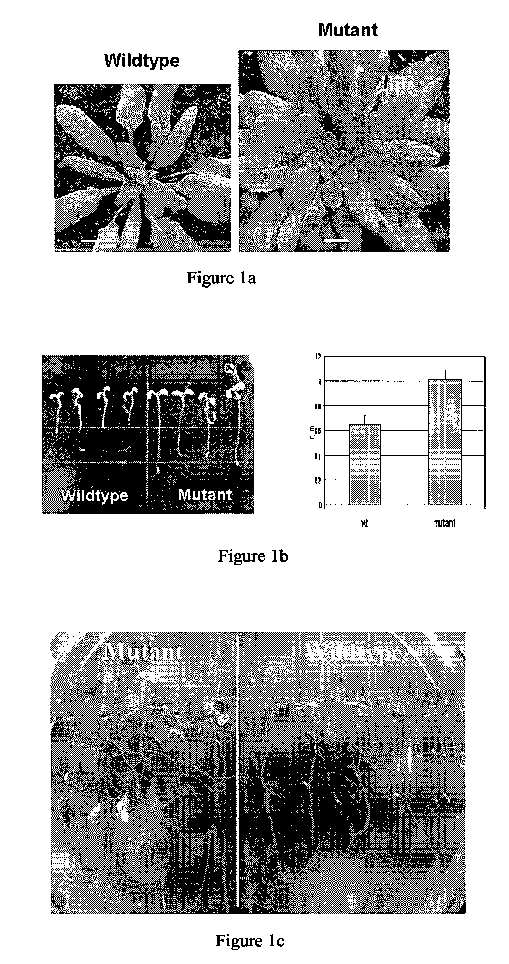

Under long daylight conditions, the mutant of 5-week old shows vigorous vegetative growth and has four times more rosette leaf numbers than that of the wild type. Its rosette is more tightly arranged and leaf petiole is shorter than that of the wild type (Figure 1a). Under short daylight conditions, the mutant shows similar morphology to the wild type but still has more leaf numbers. Under both conditions, the mutant flowers later than the wild type.

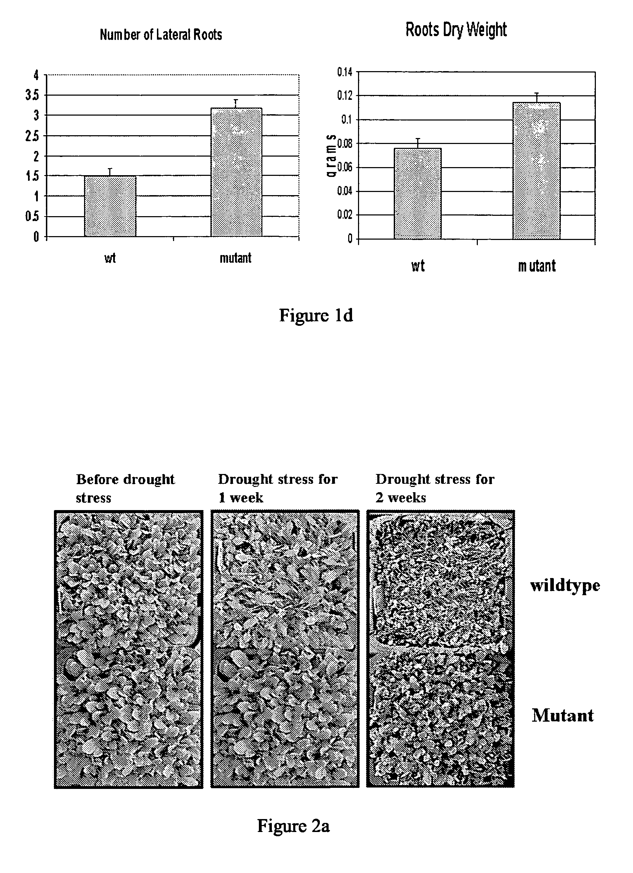

There is a distinct difference in root architecture between the mutant and the wild type. The primary root of the mutant grows faster than that of the wild type and is significantly longer than that of the wild type. The primary root length of one-week old mutant seedlings is almost twice as that of the wild type (Figure 1b). In addition, the mutant has significantly more lateral roots, twice as many as the wildtype. Root biomass of the mutant is also significantly higher than that of the wildtype (Figure 1c and Figure 1d).

Taken together, the above described mutant phenotype, especially the root architecture are just what is being sought for the agronomic improvements of many crops, horticultural plants, turf grasses, and grassland species.

### Example 2. Drought tolerance assay for the gain-of-function mutant

### 1. Comparison of the response to drought stress between the wild type and the mutant seedlings

To test the drought tolerance, two-week-old plants grown at high density with the same soil, light, temperature and water conditions were subjected to drought stress (without watering) for two weeks. Figure 2a shows that the wild type displayed wilting symptom after one-week drought stress while the mutant did not under the same conditions. After 2 weeks drought stress, the wildtype seedlings all died while the mutant seedlings still alive.

### 2. Response to drought stress of mutant T2 segregation population

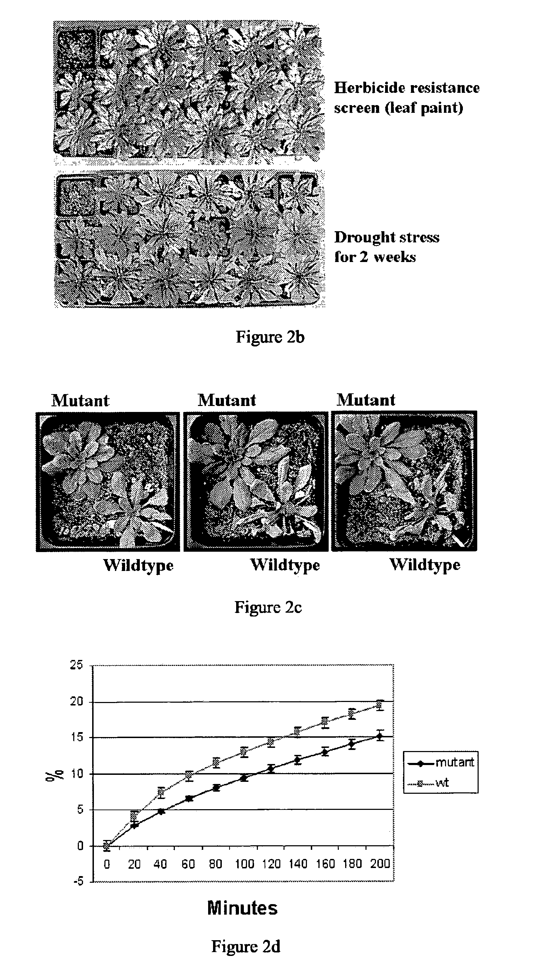

A mutant T2 segregation population is randomly cultivated in soil (under the same condition). Four weeks later, the resistance to herbicide was assayed with paintbrush method on one leaf per plant to identify the genotypes. Yellow leaf indicates the wildtype (Figure 2b). Meanwhile drought stress (without watering) was imposed for two weeks. Figure 2 shows that the drought sensitive phenotype was associated with the wildtype, thus confirming drought tolerance phenotype co-segregated with the T-DNA with herbicide resistance *Bar* gene.

### 3. Comparison of drought tolerance between the wild type and the mutant in the same pot.

A mutant and a wild type plants are cultivated in the same pot. After four weeks, drought stress was imposed for successive two weeks to observe drought stress symptom (wilting). Fig2c shows significantly improved tolerance of mutant to drought stress.

### 4. Comparison of water loss rate of detached leaves between the mutant and the wild type.

The mutant and the wild type are grown in the same tray. After four weeks, rosette leaves were detached and their water loss rate determined in the air by weighing fresh weight every 20 minutes. Water loss rate is expressed as % = (initial weight - weight at a specific time point) x 100/initial weight. Figure 2d shows the water loss rate of the wild type leaf is faster than that of the mutant leaf. This experiment indicates that the decrease of water loss rate might have contributed to the drought tolerance of the mutant.

### Example 3. Response of the mutant to salt stress in contrast to the wildtype (no part of the present invention).

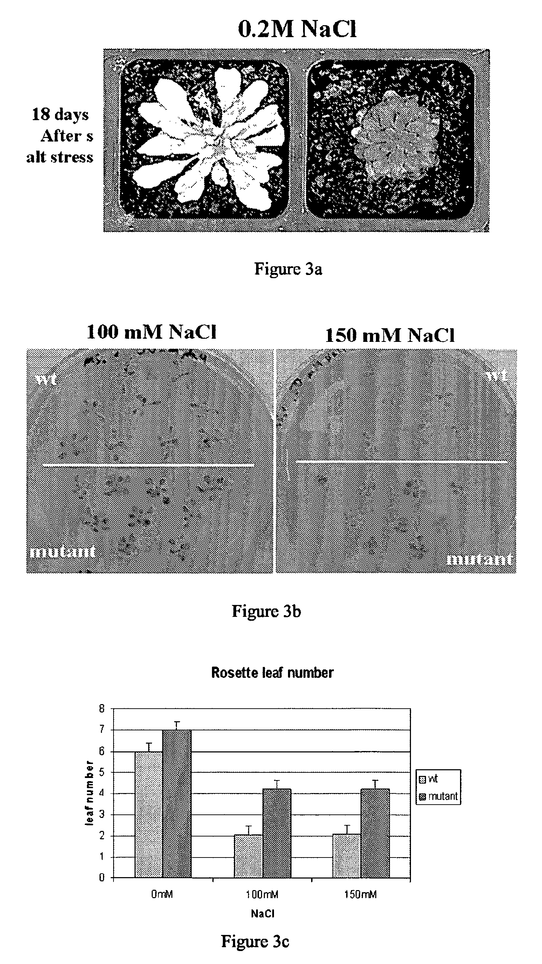

The mutant and the wild type are cultivated in the same tray under same growth conditions. Four weeks later salt stress was imposed by adding 0.2M NaCl solution. After 18 days, the salt stress phenotype could be seen in Figure 3a where the wild type died (the left one) and the mutant continues to grow (the right one). This demonstrates that in addition to drought tolerance, the mutant also has improved tolerance to salt stress.

The mutant and the wild type seeds were germinated on MS medium without salt. One week later, the seedlings were transferred to the medium containing 100 mM and 150mM NaCl and let them continue to grow. The leaf number (as an indicator of continued growth under salt stress) was counted. Fig3b and 3c show that leaf number of the mutant is higher than that of the wild type under salt stress. In Figure 3b and 3c, wt means wild type.

### Example 4. Response of the mutant to oxidation stress compared with the wildtype (no part of the present invention).

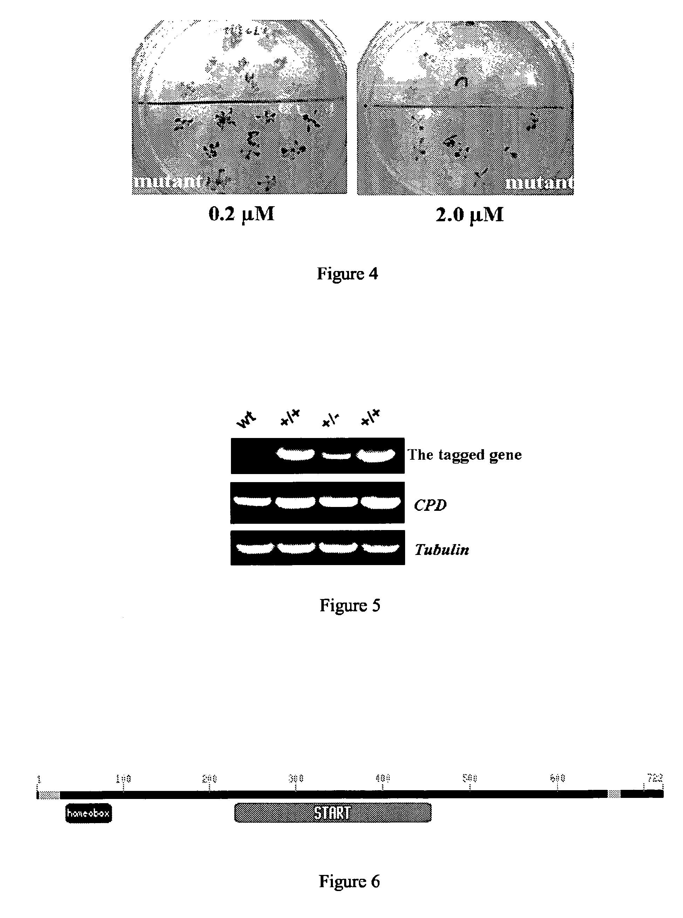

The mutant and the wild type seeds were germinated on MS medium without paraquat. One week later, the seedlings were transferred to the medium containing 0.2mM and 2.0mM paraquat and let them continue to grow for two weeks. The leaf number was counted. As shown in Fig4, mutant seedlings were more paraquat-tolerant than the wild type which became bleached in the same medium.

### Example 5. The cloning and expression analysis of the tagged gene (AtHD/START1).

The step 2 of example 2 shows that the drought tolerance phenotype co-segregated with the *BAR* gene, which strongly suggests that the enhanced stress tolerance phenotype is related to the T-DNA tagging and gives sufficient proof to clone the tagged gene.

### 1. The cloning of the T-DNA tagged gene.

First, a southern blotting experiment using *BAR* gene as probe was performed as described (Xiang et al., 1997 , DNA-binding properties, genomic organization and expression pattern of TGA6, a new member of the TGA family of bZIP transcription factors in Arabidopsis thaliana. Plant Mol Biol. 34:403-15.) and identified that the T-DNA in the genome was a single copy. Then, the plasmid rescue method was used to isolate the T-DNA insertion site as described (Weigel et al., 2000, Activation tagging in Arabidopsis. Plant Physiol. 122:1003-13). The tagged gene was identified by sequencing the T-DNA insertion junction. The nucleotide sequence of the tagged gene is shown in SEQ ID Nº : 1 in the sequence listing, and the amino acid residue sequence encoded by the gene is shown in SEQ ID Nº : 2 in the sequence listing. The gene is named as *AtHD*/*START1.*

Blast search result shows that the gene (At1g73360) is located on chromosome 1. The T-DNA was inserted in the 5' regulatory region of the gene, immediate downstream of 4 enhancers, confirming that the mutant is a true activation tagging mutant.

The blast search result with the AtHD/START1 sequence shows that there are at least 17 members in this gene family in *Arabidopsis thaliana* genome. The homology of amino acid residue sequence between *Arabidopsis thaliana* AtHD/START1 (At1g73360) and its family members are At1g17920 (80%), At4g21750 (66%), At4g04890 (66%), At1g05230 (65%), At3g61150 (65%), At4g00730 (63%), At5g52170 (60%), At5g46880 (60%), At2g32370 (58%), At3g03260 (58%), At4g17710 (58%), At5g17320 (57%), At1g79840 (57%), At1g34650 (57%), At4g25530 (51%), and At5g07260 (46%), respectively. Two structural domains, HD and START, are found as shown in Figure 6. HD is a DNA specific-binding domain and START is a hypothetical ligand binding domain.

### 2. The expression analysis of AtHD/START1

RT-PCR reactions were performed to analyze the transcript levels of *AtHD*/*START1* in rosettes of T2 mutant progeny as follows: the total RNA was extracted with Trizol reagent (INVITROGEN), the template of reverse-transcription reaction was 1µg total RNA and the primer was d(T)₂₅. 200 units SUPERSCRIPT II Reverse Transcriptase (INVITROGEN) were used for each reverse transcription reaction as described by the instruction manual. The sequence of primer for RT reaction was designed according to the sequence of *AtHD*/*START1.* The 5' end primer is 5'-atgagtttcgtcgtcggcgt-3'and the 3' end primer is 5'-tcaagctgtagttgaagctgt-3'. The template of PCR reaction was 1µl of RT reaction and others followed the protocol of ExTaq polymerase of Takara Corp. The annealing temperature was 56°C and the extension time was 100 seconds. The PCR reaction was programmed for 30 cycles. The result shown in Figure 5 demonstrates that *AtHD*/*START1* (marked gene) was not detectable in the wild type *Arabidopsis*, while highly expressed in the leaves of both homozygous (+/+) and heterozygous (+/-) mutant. The expression of the gene was totally correlated with stress tolerance phenotype, which not only confirmed that the mutation was indeed a functional activation mutation but also strongly suggested that the overexpression of the gene was the direct cause of the enhanced stress tolerance phenotype.

### Example 6: Recapitulation of the stress tolerance phenotype by overexpression of AIUDISTARTI cDNA in the wild type

The *AtHD*/*START1* cDNA was first cloned into vector pGEM-Teasy (T-A cloning). Then the *AtHD*/*START1* cDNA fragment was cut out with restriction endonuclease SacI and Sall and ligated to vector pCB302-3 digested with SpeI and SstI restriction site with the help of SacII/SpeI and SstI/SalI linkers (Xiang et al., 1999. A mini binary vector series for plant transformation. Plant Mol Biol. 40:711-7.). The resultant construct was transformed into Arabidopsis as described (Xiang et al., 2001. The biological functions of glutathione revisited in Arabidopsis transgenic plants with altered glutathione levels. Plant Physiol. 126:564-74).

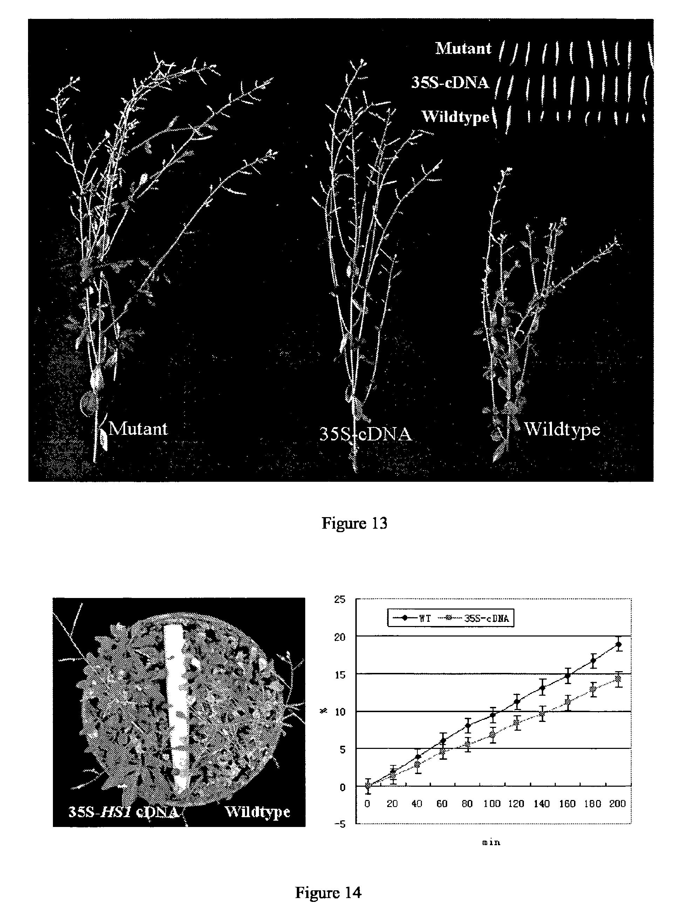

To test drought tolerance in soil, the 35S-cDNA overexpression transformant and the wild type were grown in the same pot. Results in Figure 14 show the 3-week old 35S-cDNA overexpression transformant (left) and the wild type plants (right) after drought stress for 2 weeks. The result shows that the 35S-cDNA transformants are more drought tolerant than the wild type.

Recapitulation of the mutant phenotype is one of the most important standards to determine if a gene can be used in practice or not. The experiments described above show that the improved drought stress tolerance phenotype can be recapitulated by expressing *AtHD*/*START1* cDNA, implicating the great potential of this stress tolerance gene for crop improvement.

### Industrial application

The invention can not only be used to improve the tolerance of crops but also is very important to basic studies of plant stress biology.

### Sequence Listing

<160> 2
<210> 1
   <211> 2169
   <212> DNA
   <213> *Arabidopsis thaliana*
<400> 1
   atgagtttcg tcgtcggcgt cggcggaagt ggtagtggaa gcggcggaga cggtggtggt 60
   agtcatcatc acgacggctc tgaaactgat aggaagaaga aacgttacca tcgtcacacc 120
   gctcaacaga ttcaacgcct tgaatcgagt ttcaaggagt gtcctcatcc agatgagaaa 180
   cagaggaacc agcttagcag agaattgggt ttggctccaa gacaaatcaa gttctggttt 240
   cagaacagaa gaactcagct taaggctcaa catgagagag cagataatag tgcactaaag 300
   gcagagaatg ataaaattcg ttgcgaaaac attgctatta gagaagctct caagcatgct 360
   atatgtccta actgtggagg tcctcctgtt agtgaagatc cttactttga tgaacaaaag 420
   cttcggattg aaaatgcaca ccttagagaa gagcttgaaa gaatgtctac cattgcatca 480
   aagtacatgg gaagaccgat atcgcaactc tctacgctac atccaatgca catctcaccg 540
   ttggatttgt caatgactag tttaactggt tgtggacctt ttggtcatgg tccttcactc 600
   gattttgatc ttcttccagg aagttctatg gctgttggtc ctaataataa tctgcaatct 660
   cagcctaact tggctatatc agacatggat aagcctatta tgaccggcat tgctttgact 720
   gcaatggaag aattgctcag gcttcttcag acaaatgaac ctctatggac aagaacagat 780
   ggctgcagag acattctcaa tcttggtagc tatgagaatg ttttcccaag atcaagtaac 840
   cgagggaaga accagaactt tcgagtcgaa gcatcaaggt cttctggtat tgtcttcatg 900
   aatgctatgg cacttgtcga catgttcatg gattgtgtca agtggacaga actctttccc 960
   tctatcattg cagcttctaa aacacttgca gtgatttctt caggaatggg aggtacccat 1020
   gagggtgcat tgcatttgtt gtatgaagaa atggaagtgc tttcgccttt agtagcaaca 1080
   cgcgaattct gcgagctacg ctattgtcaa cagactgaac aaggaagctg gatagttgta 1140
   aacgtctcat atgatcttcc tcagtttgtt tctcactctc agtcctatag atttccatct 1200
   ggatgcttga ttcaggatat gcccaatgga tattccaagg ttacttgggt tgaacatatt 1260
   gaaactgaag aaaaagaact ggttcatgag ctatacagag agattattca cagagggatt 1320
   gcttttgggg ctgatcgttg ggttaccact ctccagagaa tgtgtgaaag atttgcttct 1380
   ctatcggtac cagcgtcttc atctcgtgat ctcggtggag tgattctatc accggaaggg 1440
   aagagaagca tgatgagact tgctcagagg atgatcagca actactgttt aagtgtcagc 1500
   agatccaaca acacacgctc aaccgttgtt tcggaactga acgaagttgg aatccgtgtg 1560
   actgcacata agagccctga accaaacggc acagtcctat gtgcagccac cactttctgg 1620
   cttcccaatt ctcctcaaaa tgtcttcaat ttcctcaaag acgaaagaac ccgtcctcag 1680
   tgggatgttc tttcaaacgg aaacgcagtg caagaagttg ctcacatctc aaacggatca 1740
   catcctggaa actgcatatc ggttctacgt ggatccaatg caacacatag caacaacatg 1800
   cttattctgc aagaaagctc aacagactca tcaggagcat ttgtggtcta cagtccagtg 1860
   gatttagcag cattgaacat cgcaatgagc ggtgaagatc cttcttatat tcctctcttg 1920
   tcctcaggtt tcacaatctc accagatgga aatggctcaa actctgaaca aggaggagcc 1980
   tcgacgagct caggacgggc atcagctagc ggttcgttga taacggttgg gtttcagata 2040
   atggtaagca atttaccgac ggcaaaactg aatatggagt cggtggaaac ggttaataac 2100
   ctgataggaa caactgtaca tcaaattaaa accgccttga gcggtcctac agcttcaact 2160
   acagcttga 2169
<210> 2
   <211> 722
   <212> PRT
   <213> *Arabidopsis thaliana*
<400> 2
   Met Ser Phe Val Val Gly Val Gly Gly Ser Gly Ser Gly Ser Gly Gly
   1 5 10 15
   Asp Gly Gly Gly Ser His His His Asp Gly Ser Glu Thr Asp Arg Lys
   20 25 30
   Lys Lys Arg Tyr His Arg His Thr Ala Gln Gln Ile Gln Arg Leu Glu
   35 40 45
   Ser Ser Phe Lys Glu Cys Pro His Pro Asp Glu Lys Gln Arg Asn Gln
   50 55 60
   Leu Ser Arg Glu Leu Gly Leu Ala Pro Arg Gln Ile Lys Phe Trp Phe
   65 70 75 80
   Gln Asn Arg Arg Thr Gln Leu Lys Ala Gln His Glu Arg Ala Asp Asn
   85 90 95
   Ser Ala Leu Lys Ala Glu Asn Asp Lys Ile Arg Cys Glu Asn Ile Ala
   100 105 110
   Ile Arg Glu Ala Leu Lys His Ala Ile Cys Pro Asn Cys Gly Gly Pro
   115 120 125
   Pro Val Ser Glu Asp Pro Tyr Phe Asp Glu Gln Lys Leu Arg Ile Glu
   130 135 140
   Asn Ala His Leu Arg Glu Glu Leu Glu Arg Met Ser Thr Ile Ala Ser
   145 150 155 160
   Lys Tyr Met Gly Arg Pro Ile Ser Gln Leu Ser Thr Leu His Pro Met
   165 170 175
   His Ile Ser Pro Leu Asp Leu Ser Met Thr Ser Leu Thr Gly Cys Gly
   180 185 190
   Pro Phe Gly His Gly Pro Ser Leu Asp Phe Asp Leu Leu Pro Gly Ser
   195 200 205
   Ser Met Ala Val Gly Pro Asn Asn Asn Leu Gln Ser Gln Pro Asn Leu
   210 215 220
   Ala Ile Ser Asp Met Asp Lys Pro Ile Met Thr Gly Ile Ala Leu Thr
   225 230 235 240
   Ala Met Glu Glu Leu Leu Arg Leu Leu Gln Thr Asn Glu Pro Leu Trp
   245 250 255
   Thr Arg Thr Asp Gly Cys Arg Asp Ile Leu Asn Leu Gly Ser Tyr Glu
   260 265 270
   Asn Val Phe Pro Arg Ser Ser Asn Arg Gly Lys Asn Gln Asn Phe Arg
   275 280 285
   Val Glu Ala Ser Arg Ser Ser Gly Ile Val Phe Met Asn Ala Met Ala
   290 295 300
   Leu Val Asp Met Phe Met Asp Cys Val Lys Trp Thr Glu Leu Phe Pro
   305 310 315 320
   Ser Ile Ile Ala Ala Ser Lys Thr Leu Ala Val Ile Ser Ser Gly Met
   325 330 335
   Gly Gly Thr His Glu Gly Ala Leu His Leu Leu Tyr Glu Glu Met Glu
   340 345 350
   Val Leu Ser Pro Leu Val Ala Thr Arg Glu Phe Cys Glu Leu Arg Tyr
   355 360 365
   Cys Gln Gln Thr Glu Gln Gly Ser Trp Ile Val Val Asn Val Ser Tyr
   370 375 380
   Asp Leu Pro Gln Phe Val Ser His Ser Gln Ser Tyr Arg Phe Pro Ser
   385 390 395 400
   Gly Cys Leu Ile Gln Asp Met Pro Asn Gly Tyr Ser Lys Val Thr Trp
   405 410 415
   Val Glu His Ile Glu Thr Glu Glu Lys Glu Leu Val His Glu Leu Tyr
   420 425 430
   Arg Glu Ile Ile His Arg Gly Ile Ala Phe Gly Ala Asp Arg Trp Val
   435 440 445
   Thr Thr Leu Gln Arg Met Cys Glu Arg Phe Ala Ser Leu Ser Val Pro
   450 455 460
   Ala Ser Ser Ser Arg Asp Leu Gly Gly Val Ile Leu Ser Pro Glu Gly
   465 470 475 480
   Lys Arg Ser Met Met Arg Leu Ala Gln Arg Met Ile Ser Asn Tyr Cys
   485 490 495
   Leu Ser Val Ser Arg Ser Asn Asn Thr Arg Ser Thr Val Val Ser Glu
   500 505 510
   Leu Asn Glu Val Gly Ile Arg Val Thr Ala His Lys Ser Pro Glu Pro
   515 520 525
   Asn Gly Thr Val Leu Cys Ala Ala Thr Thr Phe Trp Leu Pro Asn Ser
   530 535 540
   Pro Gln Asn Val Phe Asn Phe Leu Lys Asp Glu Arg Thr Arg Pro Gln
   545 550 555 560
   Trp Asp Val Leu Ser Asn Gly Asn Ala Val Gln Glu Val Ala His Ile
   565 570 575
   Ser Asn Gly Ser His Pro Gly Asn Cys Ile Ser Val Leu Arg Gly Ser
   580 585 590
   Asn Ala Thr His Ser Asn Asn Met Leu Ile Leu Gln Glu Ser Ser Thr
   595 600 605
   Asp Ser Ser Gly Ala Phe Val Val Tyr Ser Pro Val Asp Leu Ala Ala
   610 615 620
   Leu Asn Ile Ala Met Ser Gly Glu Asp Pro Ser Tyr Ile Pro Leu Leu
   625 630 635 640
   Ser Ser Gly Phe Thr Ile Ser Pro Asp Gly Asn Gly Ser Asn Ser Glu
   645 650 655
   Gln Gly Gly Ala Ser Thr Ser Ser Gly Arg Ala Ser Ala Ser Gly Ser
   660 665 670
   Leu Ile Thr Val Gly Phe Gln Ile Met Val Ser Asn Leu Pro Thr Ala
   675 680 685
   Lys Leu Asn Met Glu Ser Val Glu Thr Val Asn Asn Leu Ile Gly Thr
   690 695 700
   Thr Val His Gln Ile Lys Thr Ala Leu Ser Gly Pro Thr Ala Ser Thr
   705 710 715 720
   Thr Ala

## Claims

1. Use of the Arabidopsis thaliana transcription factor gene *AtHD*/*START1* which includes one of the following nucleotide sequences
1) a nucleotide sequence of SEQ ID Nº:1;
2) a nucleotide sequence which encodes the amino acid sequence of SEQ ID Nº:2; and
3) a nucleotide sequence having more than 80% homology with the nucleotide sequence of SEQ ID Nº:1, which encode proteins having the same or similar biological functions
for improving drought tolerance of plants, whereby the
gene *AtHD*/*START1* is integrated in the recipient plant genome by using of expression vectors, overexpressed and transgenic plants having the gene *AtHD*/*TART1* are selected and identified by using marker genes.

2. Use according to claim1, where the transgenic plants are monocots or dicots.

3. Use according to claim 1 or 2, where the transgenic plants are selected from the group containing rice, wheat, maize, cucumber, tomato, aspen, grass or clover.

## Patentansprüche

1. Verwendung des Arabidopsis thaliana-Transkriptionsfaktorgens *AtHD*/*START1,* das eine der folgenden Nucleotidsequenzen enthält:
1) eine Nucleotidsequenz SEQ ID NO: 1;
2) eine Nucleotidsequenz, die für die Aminosäuresequenz SEQ ID NO: 2 codiert; und
3) eine Nucleotidsequenz, die mehr als 80% Homologie zu der Nucleotidsequenz SEQ ID NO: 1 aufweist, die für Proteine mit gleichen oder ähnlichen biologischen Funktionen codieren;
zur Verbesserung der Trockenheitstoleranz von Pflanzen, wobei das Gen *AtHD*/*START1* unter Verwendung von Expressionsvektoren in das Genom der Empfängerpflanze integriert und überexprimiert wird, und transgene Pflanzen, die das Gen *AtHD*/*START1* aufweisen, mit Hilfe von Markergenen selektiert und identifiziert werden.

2. Verwendung nach Anspruch 1, wobei die transgenen Pflanzen Monokotylen oder Dikotylen sind.

3. Verwendung nach Anspruch 1 oder 2, wobei die transgenen Pflanzen ausgewählt sind aus der Gruppe umfassend Reis, Weizen, Mais, Gurke, Tomate, Zitterpappel, Gras oder Klee.

## Revendications

1. Utilisation du gène du facteur de transcription *AtHD*/*START1,* chez Arabidopsis thaliana, qui comprend une des séquences nucléotidiques suivantes :
1) une séquence nucléotidique SEQ ID NO: 1,
2) une séquence nucléotidique qui code pour la séquence d'acides aminés SEQ ID NO: 2 et
3) une séquence nucléotidique ayant plus de 80 % d'homologie avec la séquence nucléotidique SEQ ID NO: 1, qui code pour des protéines ayant des fonctions biologiques identiques ou similaires,
pour améliorer la tolérance à la sécheresse des plantes, au moyen de quoi le gène *AtHD*/*START1* est intégré dans le génome de la plante receveuse en utilisant des vecteurs d'expression et surexprimé, et des plantes transgéniques ayant le gène *AtHD*/*START1* sont sélectionnées et identifiées en utilisant des gènes marqueurs.

2. Utilisation selon la revendication 1, dans laquelle les plantes transgéniques sont monocotes ou dicotes.

3. Utilisation selon la revendication 1 ou 2, dans laquelle les plantes transgéniques sont sélectionnées parmi le groupe comprenant le riz, le blé, le maïs, le concombre, la tomate, le tremble, l'herbe ou le trèfle.
